Europäisches Patentamt

European Patent Office

Office européen des brevets

(11) Publication number: **0 230 207**
**B1**

(12) **EUROPEAN PATENT SPECIFICATION**

(45) Date of publication of patent specification: 29.08.90

(51) Int. Cl.⁵: **C 07 D 401/04, A 01 N 43/64**

(21) Application number: **86810527.1**

(22) Date of filing: **19.11.86**

(54) Triazine derivative, its preparation and use.

(30) Priority: **26.11.85 US 801890**

(43) Date of publication of application:
**29.07.87 Bulletin 87/31**

(45) Publication of the grant of the patent:
**29.08.90 Bulletin 90/35**

(84) Designated Contracting States:
**AT BE CH DE ES FR GB GR IT LI NL**

(56) References cited:
**DE-A-2 221 787**
**DE-C-1 249 003**
**US-A-3 505 323**

(73) Proprietor: **SKW TROSTBERG AG**
**Postfach 1262**
**D-8223 Trostberg (DE)**

(72) Inventor: **Nickell, Louis G.**
**3730 N. Lake Shore Drive**
**Chicago Illinois 60613 (US)**
Inventor: **Stach, Leonard J.**
**274 Southcote**
**Riverside Illinois 60546 (US)**
Inventor: **Wu, Frank**
**1033 Mayfair Drive**
**Libertyville Illinois 60048 (US)**

(74) Representative: **Huber, Bernhard, Dipl.-Chem.
et al**
**Patentanwälte H. Weickmann, Dr. K. Fincke F.A.
Weickmann, B. Huber Dr. H. Liska, Dr. J. Prechtel
Möhlstrasse 22 Postfach 860 820
D-8000 München 86 (DE)**

Courier Press, Leamington Spa, England.

## Description

This invention relates to the novel compound, 3,4,5,6-tetrahydro 3-(2-chloro-pyridin-4-yl)-5-methyl-1-phenyl-1H-1,3,5-triazin-2-one.

This compound of the invention may be obtained by reacting N-(2-chloro-4-pyridinyl)-N'-phenylurea with formaldehyde and methylamine under the conditions of a Mannich reaction.

Formaldehyde is conveniently employed in the form of an aqueous solution.

The reaction is preferably effected in a solvent which is inert under the reaction conditions, e.g. methanol.

A suitable reaction temperature is from 5°C to 40°C, e.g. room temperature.

The compound of the invention may be recovered from the reaction mixture in which it is formed by working up by established procedures.

The compound of the invention has useful plant growth regulating activities: when employed at appropriate rates it may be employed to increase the size of grapes, to defoliate cotton or to combat weeds.

The term grapes as used herein means grapes from a vine of the Vitaceae family (*Vitis* generally) and includes, for example, Thompson Seedless, Perlette, Rebeir, Seedless Tokay and Interlocken.

The biological activity of the compound of the invention is illustrated by the following test.

### 1. On the grape varieties Thompson seedless and Perlette

Grape bunches are dipped in test solutions comprising 10, 50 and 100 ppm of a.i.

The test solutions are obtained by diluting a solution of 0.05 grams of a.i. in 5 ml of acetone with 495 ml of water and further dilution, with water, to the test concentration. A drop of surfactant is added to each dip solution.

The bunches of grape vine were dipped into each solution. Adjacent vines were used for each treatment. The remaining fruit on the vines was left intact. Treatments were made when most of the bunches were in the two week post-bloom period. Post-treatment evaluations were made 34 days after treatment. The size of the grape was determined on a scale of 1—5, where 1=no increase and 5=maximum increase. Similarly the phytotoxicity to the grape was determined on a scale of 1—5, where 0=no phytotoxicity and 5=dead berries, as follows:

TABLE I

| Grape | Property | Rate of application (ppm) | | | |
| | | 0 | 10 | 50 | 100 |
|---|---|---|---|---|---|
| Thompson Seedless | Size increase | 0 | 2 | 3 | 4 |
| | Phytotoxicity | 0 | 1 | 3 | 3 |
| | Weight (grams) | 1.5 | 2.1 | 2.9 | 2.2 |
| Perlette | Size increase | 1 | 2.5 | 3 | 3 |
| | Phytotoxicity | 0 | 0.5 | 1 | 2 |
| | Weight (grams) | 2.1 | 3.0 | 3.2 | 2.8 |

### 2. Cotton defoliant test

Cotton plants were grown in the greenhouse under high intensity illumination. At the seven true-leaf stage, the cotton plants were foliarly treated to run off with aqueous solutions of the test compound at concentrations of 50, 100 and 200 ppm.

Data were recorded three weeks after application of the test compound. The value for each system is a score relative to a scale of 0—10. The data represent averages for 3 plants per treatment. The value for necrosis (N) indicates a relative proportion of leaf tissue which has died. Leaf abscission (D) is based on the leaves present at the time of application of the test compound, while regrowth is described by apical meristem damage (M) and growth from axillary buds (A).

TABLE II

| Rate of application (ppm) | N | M | D | A |
|---|---|---|---|---|
| 200 | 3.3 | 8.0 | 9.3 | 4.3 |
| 100 | 4.0 | 5.0 | 8.0 | 2.7 |
| 50 | 1.7 | 0.0 | 7.3 | 0.3 |

3. Herbicidal activity
3.1 Pre-emergence control

Small greenhouse pots filled with dry soil were seeded with the various weed seeds. Twenty-four hours or less after seeding the pots were sprayed with water until the soil was wet and the test compound formulated as an aqueous emulsion of acetone solution containing emulsifiers was sprayed at eight pounds per acre on the surface of the soil.

After spraying, the soil containers were placed in the greenhouse and provided with supplementary heat as required and daily or more frequent watering. The plants were maintained under these conditions for a period of 21 days, at which time the condition of the plants and the degree of injury to the plants was rated on a scale of from 0 to 10, as follows: 0=injury; 1, 2=slight injury; 7—9=severe injury; and 10=death. The effectiveness of this compound is demonstrated by the data in Table III.

3.2 Post emergence control

The herbicidal activity of the compound of this invention was also demonstrated by experiments carried out for the post-emergence control of a variety of weeds. In these experiments the compound was formulated as an aqueous emulsion and sprayed at the indicated dosage on the foliage of the weeds that have attained a prescribed size. After spraying, the plants were placed in a greenhouse and watered daily or more frequently. Water was not applied to the foliage of the treated plants. The severity of the injury was determined 14 days after treatment and was rated on the scale of from 0 to 10 heretobefore described. The effectiveness of the compound is demonstrated by the following data:

TABLE III

| Plant | Pre-emergence rating | Post-emergence rating |
|---|---|---|
| Velvetleaf | 8 | 8 |
| Pigweed | 2 | 10 |
| Crabgrass | 8 | 7 |
| Barnyard grass | 4 | 7 |
| Yellow foxtail | 4 | 8 |

The present invention therefore also provides a method of regulating plant growth which comprises applying to the plants or their locus a plant growth regulating amount of the compound of the invention.

The amount to be applied will vary depending on the desired effect, the plant locus, plant or plant variety to be treated, mode and time of application, conditions of treatment and the like. The appropriate application rates can be determined by routine experiments by those skilled in the art, or by comparing the activity of the compound of the invention with standards for which the application rate is known, e.g. in greenhouse test.

For use as a grape size increaser the compound should be applied in a grape size increasing effective amount. Satisfactory results are in general obtained with application rates of from 5 to 300 grams, more specifically 50 to 100 grams per acre. Above 400 grams per acre the phytotoxicity is excessive.

Generally, the compound should be applied at from about 10 days post-bloom to about two weeks prior to the intended harvest time of the grapes. It is conveniently applied in the form of an aqueous solution or dispersion.

A suitable application volume is from 5 to 200 gallons per acre.

As a defoliant for cotton, the compounds is applied to the cotton plants in defoliating effective amount, e.g. as an aqueous solution preferably containing from about 50 to about 500 ppm of the compound. In order to ensure that an effective amount is applied to the cotton plant, it is applied to run off the plants. It is conveniently applied to the cotton plant from about 10 to about 30 days prior to harvest.

For combatting weeds the compound of the invention is applied to the weeds or their locus in a herbicidally effective amount. The compound can be applied before or after emergence of the weeds to be combatted.

The compound of the invention may be and preferably is employed as a plant growth regulating composition in association with agriculturally acceptable diluent(s). Suitable formulations contain 0.01% to 99% by weight of active ingredient, from 0 to 15% surfactant and 1% to 99.99% solid or liquid diluent(s). Higher ratios of surfactant to active ingredient are sometimes desirable and are achieved by incorporation into the formulation or by tank mixing. Application forms of composition generally contain between 0.01, and 25% by weight of active ingredient. Lower or higher levels of active ingredient can, of course, be present depending on the intended use and the mode of application. Concentrate forms of composition intended to be diluted before use generally contain between 2 and 90%, preferably between 10 and 80% by weight of active ingredient.

Useful formulations of the compound of the invention include dusts, granulates, suspension concentrates, wettable powders, emulsifiable concentrates and the like. They are obtained by conventional manner, e.g. by mixing the compound of the invention with the diluent(s) and optionally with other ingredients.

Alternatively, the compound of the invention may be used in micro-encapsulated form.

Agriculturally acceptable additives may be employed in the compositions to improve the performance of the active ingredient and to reduce foaming, caking and corrosion.

Surfactant as used herein means an agriculturally acceptable material which imparts emulsifiability, spreading, wetting, dispersibility or other surface-modifying properties. Examples of surfactants are sodium lignin sulphonate, lauryl sulphate, sorbitan monolaurate and polyoxyethylene oleyl ether.

In the aqueous solutions employed, it is preferred to use a surfactant. The surfactant level is generally from 0.1 to 15% by volume of the total formulation and 0.1 to 1.5% by volume is preferred.

Diluents as used herein mean a liquid or solid agriculturally acceptable material used to dilute a concentrated material to a usuable or desirable strength. For dusts or granules it can be e.g. talc, kaolin ordiamaceous earth, for liquid concentrate forms for example a hydrocarbon such as xylene or an alcohol such as isopropanol, and for liquid application forms i.a. water or diesel oil.

The compositions of this invention can also comprise other compounds having biological activity, e.g. compounds having similar or complementary herbicidal activity or compounds having antidotal, fungicidal or insecticidal activity.

The invention is further illustrated by the following example.

Example 1

Preparation of 3,4,5,6-tetrahydro-3-(2-chloropyridin-4-yl)-5-methyl-1-phenyl-1H-1,3,5-triazin-2-one

Formaldehyde (15 mole, 98% aqueous solution) was added dropwise to a mixture of N-(2-chloro-4-pyridinyl)-N'-phenylurea (3.0 grams; 0.012 mole), methylamine (15 ml, 40% aqueous solution) and methanol (20 ml) in a three-necked reaction flask equipped with stirrer, thermometer and addition funnel. This reaction mixture was stirred for twenty hours at room temperature and then evaporated by Gredwood pressure to give the desired product as a pale yellow oil (4.0 grams). This product analysed as follows:

|  | Theoretical (%) | Found (%) |
| --- | --- | --- |
| Carbon | 59.50 | 57.15 |
| Hydrogen | 4.99 | 5.56 |
| Nitrogen | 18.51 | 19.60 |

**Claims for the Contracting States: BE, CH, DE, FR, GB, GR, IT, LI, NL**

1. 3,4,5,6-tetrahydro-3-(2-chloropyridin-4-yl)-5-methyl-1-phenyl-1H-1,3,5-triazin-2-one.

2. A method of regulating plant growth which comprises applying to the plants or their locus a plant growth regulating amount of the compound of Claim 1.

3. The method of Claim 2 for increasing the size of grapes which comprises applying to the grape plants a grape size increasing effective amount of the compound of Claim 1.

4. The method of Claim 3 wherein the compound of claim is applied to the grapes from about 10 days post-bloom to about 2 weeks prior to harvest.

5. The method of Claims 3 and 4 wherein the amount of the compound of Claim 1 applied to the grape plants is from 5 to 300 grams per acre.

6. The method of Claim 2 for defoliating cotton which comprises applying to the cotton plants a defoliating effective amount of the compound of Claim 1.

7. The method of Claim 2 for combatting weeds which comprises applying to the weeds or their locus a herbicidally effective amount of the compound of Claim 1.

8. A plant growth regulating composition comprising the compound of Claim 1 and a diluent.

9. Process for preparing the compound of Claim 1, which comprises reacting N-(2-chloro-4-pyridinyl)-N'-phenylurea with formaldehyde and methylamine under the conditions of a Mannich reaction.

**Claims for the Contracting States: AT, ES**

1. Process of preparing 3,4,5,6-tetrahydro-3-(2-chloropyridin-4-yl)-5-methyl-1-phenyl-1H-1,3,5-triazin-2-one which comprises reacting N-(2-chloro-4-pyridinyl)-N'-phenyl urea with formaldehyde and methylamine under the conditions of a Mannich reaction.

2. A plant growth regulating composition comprising 3,4,5,6-tetrahydro-3-(2-chloropyridin-4-yl)-5-methyl-1-phenyl-1H-1,3,5-triazin-2-one and a diluent.

## Patentansprüche für die Vertragsstaaten: BE, CH, DE, FR, GB, GR, IT, LI, NL

1. 3,4,5,6-Tetrahydro-3-(2-chlorpyridin-4-yl)-5-methyl-1-phenyl-1H-1,3,5-triazin-2-on.

2. Verfahren zum Regulieren von Pflanzenwachstum, umfassend das Aufbringen einer das Pflanzenwachstum regulierenden Menge der Verbindung nach Anspruch 1 auf die Pflanzen oder ihren Standort.

3. Verfahren nach Anspruch 2 zur Steigerung der Größe von Trauben, umfassend das Aufbringen einer für die Größensteigerung von Trauben wirksamen Menge der Verbindung nach Anspruch 1 auf die Traubenpflanzen.

4. Verfahren nach Anspruch 3, worin die Verbindung nach Anspruch 1 auf die Trauben von ungefähr 10 Tagen nach der Blüte bis ungefähr 2 Wochen vor der Ernte aufgebracht wird.

5. Verfahren nach Anspruch 3 und 4, worin die Menge der Verbindung nach Anspruch 1, die auf die Traubenpflanzen aufgebracht wird, von 5 bis 300 g pro Acre ist.

6. Verfahren nach Anspruch 2 zur Entlaubung von Baumwolle, umfassend das Aufbringen einer für die Entlaubung wirksamen Menge der Verbindung nach Anspruch 1 auf die Baumwollpflanzen.

7. Verfahren nach Anspruch 2 zur Bekämpfung von Unkräutern, umfassend das Aufbringen einer herbizid wirksamen Menge der Verbindung nach Anspruch 1 auf die Unkräuter oder ihren Standort.

8. Das Pflanzenwachstum regulierende Zusammensetzung, umfassend die Verbindung nach Anspruch 1 und ein Verdünnungsmittel.

9. Verfahren zur Herstellung der Verbindung nach Anspruch 1, umfassend das Umsetzen von N-(2-Chlor-4-pyridinyl)-N'-phenylharnstoff mit Formaldehyd und Methylamin unter den Bedingungen einer Mannich-Reaktion.

## Patentansprüche für die Vertragsstaaten: AT, ES

1. Verfahren zur Herstellung von 3,4,5,6-Tetrahydro-3-(2-chlorpyridin-4-yl)-5-methyl-1-phenyl-1H-1,3,5-triazin-2-on, umfassend das Umsetzen von N-(2-Chlor-4-pyridinyl)-N'-phenylharnstoff mit Formaldehyd und Methylamin unter den Bedingungen einer Mannich-Reaktion.

2. Das Pflanzenwachstum regulierende Zusammensetzung, umfassend 3,4,5,6-Tetrahydro-3-(2-chlorpyridin-4-yl)-5-methyl-1-phenyl-1H-1,3,5-triazin-2-on und ein Verdünnungsmittel.

## Revendications pour les Etats Contractants: BE, CH, DE, FR, GB, GR, IT, LI, NL

1. 3,4,5,6-tétrahydro-3-(2-chloropyridine-4-yl)-5-méthyl-1-phényl-1H-1,3,5-triazine-2-one.

2. Un procédé de régulation de la croissance des plantes qui comprend l'application aux plantes ou à leurs sites d'une quantité assurant la régulation de la croissance des plantes du composé de la revendication 1.

3. Le procédé de la revendication 2 pour accroître la taille des raisins, qui comprend l'application aux vignes d'une quantité efficace pour accroître la taille des raisins du composé de la revendication 1.

4. Le procédé de la revendication 3, dans lequel le composé de la revendication 1 est appliqué aux raisins environ 10 jours après la floraison et environ 2 semaines avant la récolte.

5. Le procédé des revendications 3 et 4, dans lequel la quantité du composé de la revendication 1 à appliquer aux vignes est 5 à 300 g pour 0,40 ha (1 acre).

6. Le procédé de la revendication 2 pour défolier le cotonnier, qui comprend l'application au cotonnier d'une quantité défoliante efficace du composé de la revendication 1.

7. Le procédé de la revendication 2 pour lutter contre les mauvaises herbes, qui comprend l'application aux mauvaises herbes ou à leurs sites d'une quantité herbicide efficace du composé de la revendication 1.

8. Une composition de régulation de la croissance des plantes comprenant le composé de la revendication 1 et un diluant.

9. Procédé pour préparer le composé de la revendication 1, qui comprend la réaction de la N-(2-chloro-4-pyridinyl)-N'-phénylurée avec le formaldéhyde et la méthylamine dans les conditions d'une réaction de Mannich.

## Revendications pour les Etats Contractants: AT, ES

1. Procédé pour préparer la 3,4,5,6-tétrahydro-3-(2-chloropyridine-4-yl)-5-méthyl-1-phényl-1H-1,3,5-triazine-2-one, qui comprend la réaction de la N-(2-chloro-4-pyridinyl)-N'-phénylurée avec le formaldéhyde et la méthylamine dans les conditions d'une réaction de Mannich.

2. Composition de régulation de la croissance des plantes comprenant de la 3,4,5,6-tétrahydro-3-(2-chloropyridine-4-yl)-5-méthyl-1-phényl-1H-1,3,5-triazine-2-one et un diluant.